# EUROPEAN PATENT APPLICATION

(11) **EP 4 825 398 A1**
(43) Date of publication of application: **21.10.2026**
(21) Application number: 25744751.6
(22) Date of filing: 24.01.2025
(51) Int. Cl.: A61K 45/06, A61P 35/00

(54) **PHARMACEUTICAL COMPOSITION CONTAINING PRMT5 INHIBITOR AND CDK4/6 INHIBITOR**

(30) Priority: 26.01.2024 CN 202410116110; 04.03.2024 CN 202410245723
(71) Applicant: Apeiron Therapeutics (Hong Kong) Ltd., Hong Kong 999077 (HK)
(72) Inventor: MAO, Weifeng, Shanghai 201210 (CN); YAO, Bing, Shanghai 201210 (CN); ASWAD, Fred Jullien, Shanghai 201210 (CN); LI, Mingxi, Shanghai 201210 (CN); GU, Xiaohui, Shanghai 201210 (CN); JOSEPH, James David, Shanghai 201210 (CN)
(74) Representative: Bayramoglu et al.
(86) International application number: PCT/CN2025/074948
(87) International publication number: WO 2025/157288

(57) **Abstract**

A pharmaceutical composition comprising protein arginine methyltransferase 5 (PRMT5) and cyclin-dependent kinase 4 and 6 (CDK4/6) inhibitors. The pharmaceutical composition can be used for treating various cancers, comprising solid tumors. The combined product can be used for treating any number of diseases associated with PRMT5 and/or CDK4/6.

## Description

### TECHNICAL FIELD

The present invention belongs to the field of medicine, and specifically relates to a pharmaceutical composition comprising a PRMT5 inhibitor and a CDK4/6 inhibitor.

### BACKGROUND ART

Cancer is among the leading causes of death worldwide. Popular treatments, such as chemotherapy and immunotherapy, are limited in that their cytotoxic effects are not restricted to cancer cells and can also cause adverse effects in normal tissues.

Homozygous deletions of p16/CDKN2a is prevalent in cancers, and these mutations often involve co-deletion of adjacent genes, including the gene encoding methylthioadenosine phosphorylase (MTAP). It is estimated that homozygous deletion of the MTAP gene occurs in approximately 15% of human cancers (Firestone and Schramm (2017) J. Am. Chem Soc. 139(39):13754-13760).

PRMT5 is a type II arginine methyltransferase that regulates important cellular functions, including cell cycle progression, apoptosis and DNA damage response, by symmetrically dimethylating proteins involved in transcription and signal transduction. However, data from genome-wide genetic perturbation screens using shRNA revealed a selective requirement for PRMT5 activity in MTAP-deleted cancer cell lines (Kruykov *et al.,* 2016; Marjon *et al.,* 2016, Markarov *et al.,* 2016). The accumulation of MTA caused by MTAP deletion in these cell lines partially inhibits PRMT5, making these cells selectively sensitive to additional PRMT5 inhibition.

Certain PRMT5 inhibitors have been developed, but they do not show selectivity for MTAP-deleted cancer cell lines. This lack of selectivity could be explained by the inhibitors' mechanism of action, as they are either SAM-noncompetitive or SAM-competitive inhibitors and therefore independent of MTAP (Kruykov *et al.,* 2016; Marjon *et al.,* 2016; Markarov *et al.,* 2016).

By using inhibitors that bind PRMT5 in an MTA-noncompetitive or MTA-cooperative manner, selectivity for MTAP-deleted/MTA-accumulating cells can be improved. PRMT5 inhibitors that bind in an MTA-noncompetitive or MTA-cooperative manner will exhibit increased binding to PRMT5 in the presence of MTA compared to the binding of the same inhibitor in the absence of MTA. Consequently, such inhibitors will bind with significantly greater potency in the presence of high concentrations of MTA and, therefore, lead to preferential inhibition of PRMT5 in MTA-accumulating cells relative to normal cells.

Despite many recent advances in cancer treatment, there remains a need for more effective and/or enhanced therapies for individuals affected by cancers.

### SUMMARY OF INVENTION

In order to solve the technical problem of the present invention, the present invention provides a pharmaceutical composition comprising a PRMT5 inhibitor having a first active substance and a CDK4/6 inhibitor compound having a second active substance, wherein the PRMT5 inhibitor having the first active substance has the structure of formula (I) or formula (II): 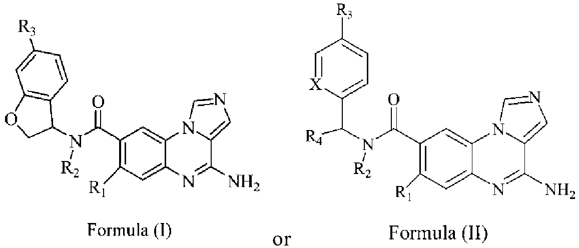 wherein, in formula (I) or formula (II),
R₁ is selected from H, halogen, C₁-C₆ alkyl, C₁-C₆ haloalkyl, and CN;
R₂ is selected from H, C₁-C₆ alkyl, C₁-C₆ haloalkyl, and C₃-C₆ cycloalkyl;
R₃ represents H, halogen, C₁-C₆ alkyl, C₁-C₆ haloalkyl, C₁-C₆ haloalkoxy, or SF₅;
in formula (II), R₄ represents hydrogen or C₁-C₆ alkyl; X represents CR₅ or N;
wherein, R₅ represents hydrogen, halogen, C₁-C₆ alkyl, C₁-C₆ haloalkyl, hydroxyl, -NH₂, or CN.

In a preferred embodiment of the present disclosure, in the structure of formula (I) or formula (II):
R₁ is selected from hydrogen, halogen, C₁-C₆ alkyl, and C₁-C₆ haloalkyl;
R₂ is selected from hydrogen, C₁-C₆ alkyl, C₁-C₆ haloalkyl, and C₃-C₆ cycloalkyl;
R₃ represents hydrogen, halogen, C₁-C₆ haloalkyl, C₁-C₆ haloalkoxy, or SF₅;
R₄ represents hydrogen or methyl;
in formula (II), X represents CH or N.

In a preferred embodiment of the present disclosure, R₁ is selected from hydrogen or fluorine.

In a preferred embodiment of the present disclosure, R₂ is selected from cyclopropyl or methyl.

In a preferred embodiment of the present disclosure, R₃ represents CF₃.

In a preferred embodiment of the present disclosure, R₄ represents hydrogen or methyl.

In a preferred embodiment of the present disclosure, X represents N.

In a preferred embodiment of the present disclosure, the PRMT5 inhibitor having the first active substance is selected from the following compounds or any combination thereof:

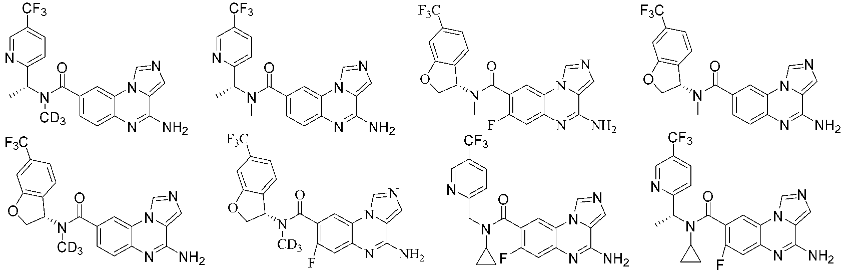

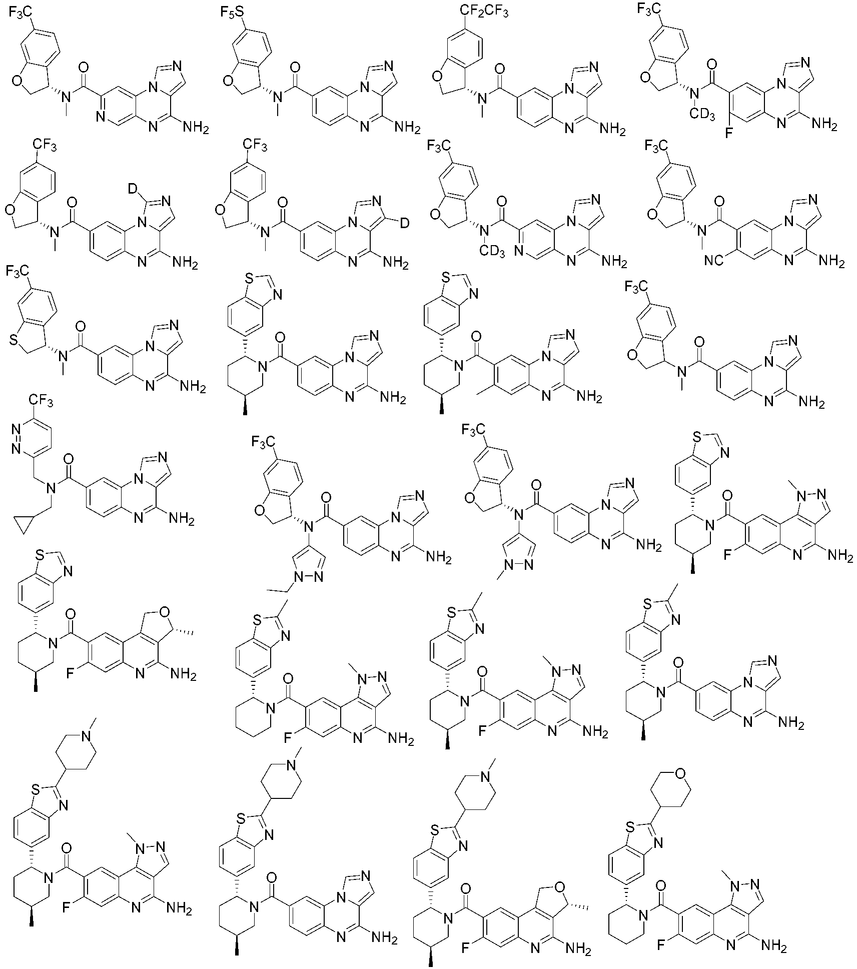

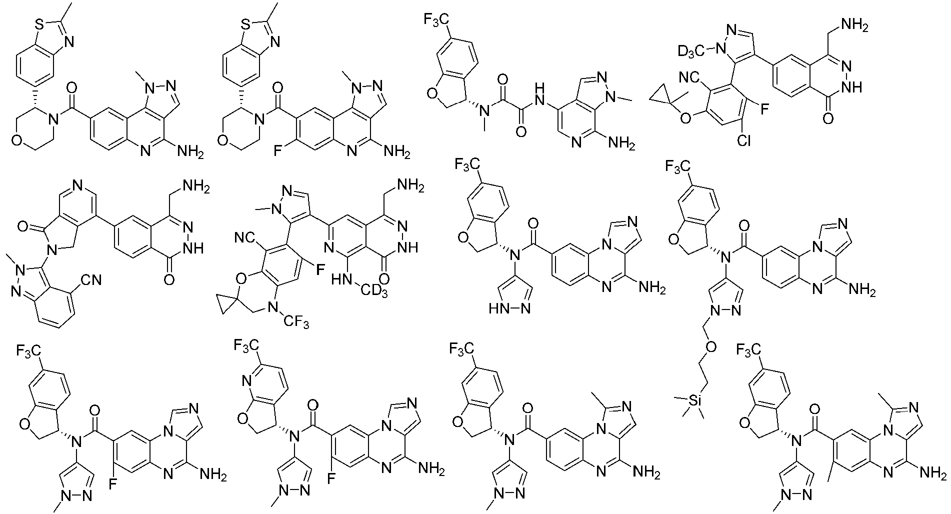

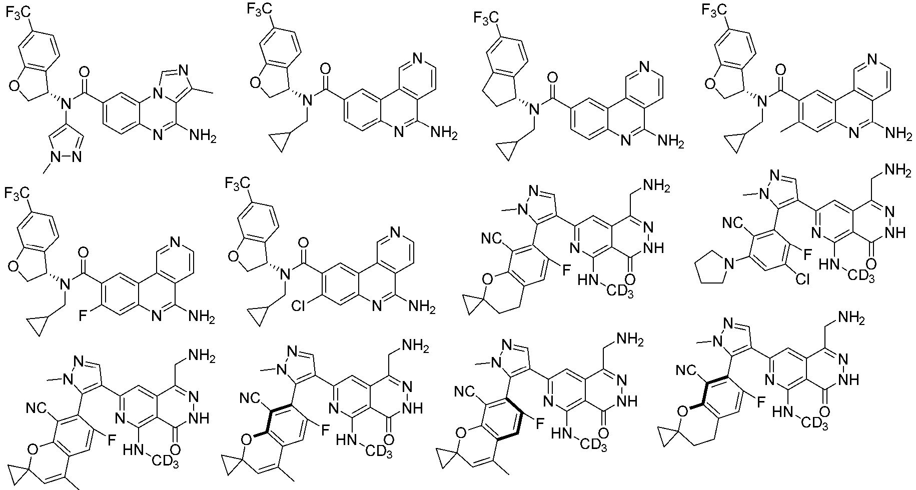

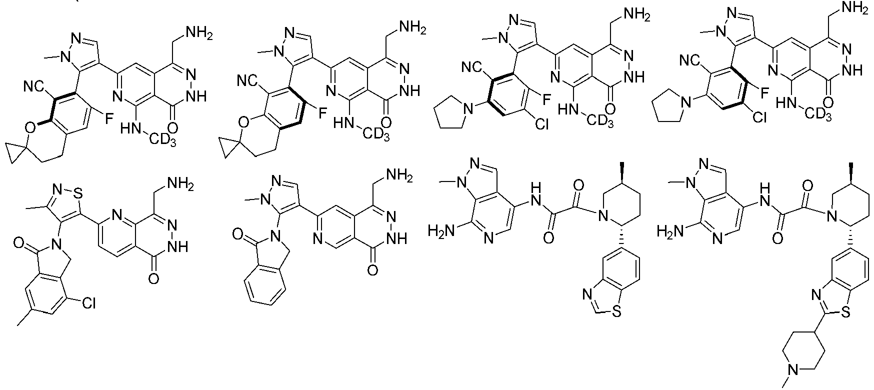

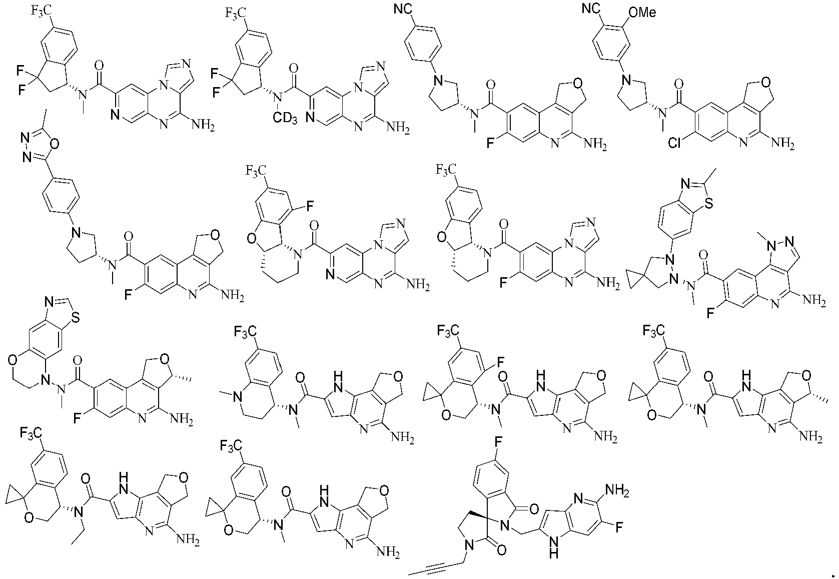

In a preferred embodiment of the present disclosure, the CDK4/6 inhibitor having the second active substance is selected from any one of the following compounds or a pharmaceutically acceptable salt thereof: Palbociclib (Ibrance^{®}), Abemaciclib (Verzenio^{®}), Ribociclib (Kisqali^{®}), Trilaciclib (Cosela^{™}), PF-06873600 (CAS: 2185857-97-8) or any combination thereof.

In a preferred embodiment of the present disclosure, the CDK4/6 inhibitor having the second active substance is selected from Palbociclib (Ibrance^{®}), Abemaciclib (Verzenio^{®}), Ribociclib (Kisqali^{®}), Trilaciclib (Cosela^{™}) or a pharmaceutically acceptable salt thereof.

In a preferred embodiment of the present disclosure, the CDK4/6 inhibitor having the second active substance is selected from Abemaciclib (Verzenio^{®}) or a pharmaceutically acceptable salt thereof.

In addition, the present invention further provides a method for treating cancer or a tumor, comprising administering the pharmaceutical composition of the present invention to an individual in need.

In a preferred embodiment of the present disclosure, the tumor or cancer is selected from: glioblastoma multiforme, brain cancer, prostate cancer, pancreatic cancer, mantle cell lymphoma, non-Hodgkin's lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, cholangiocarcinoma, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, cutaneous squamous cell carcinoma, nasopharyngeal carcinoma, neuroblastoma, endometrial cancer, head and neck cancer, and cervical cancer.

As will be understood by those of ordinary skill in the art, in any embodiment disclosed herein, any feasible combination of a compound of formula (I) or a pharmaceutically acceptable salt, solvate or prodrug thereof and a compound of formula (II) or a pharmaceutically acceptable salt, solvate or prodrug thereof is included in the present invention, provided that such combination is capable of producing some synergistic effect in the treatment of a subject in need.

When any compound is used in the present invention, it includes any pharmaceutically acceptable form thereof, including but not limited to isomers, tautomers, salts, solvates, polymorphs, prodrugs, and the like. It should be understood that the term "compound" includes any and all such forms, whether explicitly stated or not, although sometimes only certain terms are explicitly stated, such as "salt" and "prodrug".

Unless otherwise explicitly defined, all terms used herein have the ordinary meaning as would be interpreted or understood by one of ordinary skill in the art.

As used herein, the term "a", "an" or "the" refers to both the singular and plural forms. Generally, when a singular or plural form of a noun is used, it refers to both the singular and plural forms of the noun.

When the term "about" is applied to a parameter, it means that the parameter can vary within ±10%, preferably within ±5%, including any number from the lower limit to the upper limit. When the term "about" is applied to a range, it applies to both the lower and upper limits of the range. As will be understood by those skilled in the art, when a parameter is not critical, a number is generally given for illustrative purposes only and is not limiting.

"Alkoxy" refers to a group - OR, where R is alkyl as defined herein. Representative examples include methoxy, ethoxy, propoxy, isopropoxy, sec-butoxy, tert-butoxy, and the like.

"Alkyl" refers to a group derived from a straight or branched chain saturated hydrocarbon by removing a hydrogen from one of the saturated carbons. Alkyl preferably contains 1 to 8 carbon atoms, sometimes preferably 1 to 6 carbon atoms, and sometimes even more preferably 1 to 4 carbon atoms. Representative examples of alkyl include, but are not limited to, methyl, ethyl, n-propyl, isopropyl, n-butyl, sec-butyl, tert-butyl, pentyl, hexyl, and the like. "Lower alkyl," "lower alkoxy," or "lower haloalkyl" refers to an alkyl or alkyl moiety having one to four, sometimes preferably one to three or one to two carbon atoms.

As used herein, the term "cyano" refers to - CN.

As used herein, the term "cycloalkyl" refers to a group derived from a monocyclic saturated carbocyclic ring by removing a hydrogen atom from the saturated carbocyclic ring, preferably having 3 to 8, more preferably 3 to 6 carbon atoms. Representative examples of cycloalkyl include, but are not limited to, cyclopropyl, cyclopentyl, and cyclohexyl.

As used herein, the terms "halo" and "halogen" refer to F, Cl, Br, or I.

As used herein, the term "haloalkyl" refers to an alkyl group substituted with at least one halogen atom. A haloalkyl group can be an alkyl group in which all hydrogen atoms are substituted with halogens. Representative examples of haloalkyl include, but are not limited to, trifluoromethyl, fluoromethyl, difluoromethyl, bromomethyl, 1- chloroethyl, perchloroethyl, 2- fluoroethyl, and the like.

As used herein, the term "heterocyclyl" refers to a 3 to 10-membered monocyclic or bicyclic non-aromatic group containing one or more, preferably 1 to 3, heteroatoms independently selected from nitrogen (N), oxygen and sulfur (S, S(O) or S(O)2) in the non-aromatic ring. The heterocyclyl of the present invention can be attached to the parent molecular moiety through a carbon atom or a nitrogen atom in the group. The heterocyclyl group can be saturated or unsaturated, for example, containing one or more double bonds in the ring. Unless otherwise stated, the valence of the group can be located on any atom of any ring within the group where the valence rules permit. Examples may include, but are not limited to, azetidinyl, pyrrolidinyl, 2- oxopyrrolidinyl, 2,5- dihydro- 1H- pyrrolyl, piperidinyl, 4- piperidinyl, morpholinyl, piperazinyl, 2- oxopiperazinyl, tetrahydropyranyl, tetrahydrofuranyl, 2- oxopiperidinyl, thiomorpholinyl, pyrazolidinyl, imidazolinyl, imidazolidinyl, and the like.

When any group, such as "cycloalkyl" or "heterocyclyl", is referred to as "substituted or unsubstituted" or "optionally substituted", unless otherwise specified, it means that the group is substituted or unsubstituted by 1 to 5, sometimes preferably 1 to 3 or 1 to 2 substituents independently selected from halogen, C1- C6 alkyl, C1- C6 alkoxy, C1- C6 haloalkyl and cyano. As used herein, the term "solvate" refers to a physical association of a compound of the present invention with one or more, preferably one to three, solvent molecules (whether organic or inorganic). This physical association includes hydrogen bonding. In some cases, the solvate is capable of isolation, for example when one or more, preferably one to three, solvent molecules are incorporated into the crystal lattice of a crystalline solid. Exemplary solvates include, but are not limited to, hydrates, ethanolates, methanolates, and isopropanolates. Solvation methods are generally known in the art.

"Prodrugs" refer to compounds that can be converted *in vivo* to produce the active parent compound under physiological conditions, such as by hydrolysis in the blood. Common examples include, but are not limited to, ester and amide forms of compounds having an active form with a carboxylic acid moiety. Amides and esters of the compound of the present invention can be prepared according to conventional methods. In particular, in the present invention, prodrugs can also be formed by acylating the amino group or nitrogen atom in the heterocyclyl ring structure, where the acyl group can be hydrolyzed *in vivo.* Such acyl groups include, but are not limited to, C1- C6 acyl, preferably C1- C4 acyl, more preferably C1- C2 formyl or acetyl, or benzoyl.

As used herein, the term "subject" refers to a human or other mammal, such as a monkey, dog, cat, horse and the like. The term is intended to encompass and is sometimes interchangeable with "patient".

As used herein, the term "administering" or "administration" refers to providing a compound or pharmaceutical composition to a subject having or at risk for a disease or condition to be treated or prevented.

Any route of administration is suitable for the present invention. In one embodiment, the compound of the present invention can be administered to a subject in a solid dosage form such as tablets, capsules, and the like. In one embodiment, the compound of the present invention can be administered to a subject by intravenous injection. In another embodiment, the compound of the present invention can be administered to a subject by any other suitable systemic delivery method, such as oral, parenteral, intranasal, sublingual, rectal or transdermal administration.

As used herein, the term "therapeutically effective amount" refers to the amount of a compound or composition that will elicit the desired or expected biological or medical response in a subject that is being sought by a physician, veterinarian, or researcher. The therapeutically effective amount of a compound and a particular pharmaceutically acceptable carrier will vary depending on, for example, age, weight, sex of the subject, route of administration, and the disease or condition being treated.

The term "pharmaceutically acceptable" when used before a compound, salt, prodrug, composition, or carrier means that such compound, salt, prodrug, composition, or carrier is suitable for administration to a subject for treatment without causing intolerable side effects to the subject considering the desired treatment.

As used herein, the term "pharmaceutically acceptable carrier" refers to a substance that is compatible with the compounds used in the present invention and can be used to administer the compounds in the methods of the present invention, and is preferably non-toxic, or inert and pharmaceutically acceptable. Pharmaceutically acceptable carriers can be solid, liquid or gaseous substances, including any and all dry powders, solvents, dispersion media, coatings, antibacterial and antifungal agents, isotonic agents, absorption delaying agents, and the like. Examples of such carriers include oils such as corn oil, buffers such as phosphate buffered saline (PBS), saline, polyethylene glycol, glycerol, polypropylene glycol, dimethyl sulfoxide, amides such as dimethylacetamide, proteins such as albumin, detergents such as Tween 80, monosaccharides and oligosaccharides such as glucose, lactose, cyclodextrins, starch, and the like.

As described herein, some embodiments of the compound of the present invention can contain basic functional groups, such as amino or alkylamino, and therefore can form pharmaceutically acceptable salts with pharmaceutically acceptable acids. In this respect, the term "pharmaceutically acceptable salt" refers to relatively nontoxic inorganic and organic acid addition salts of the compound of the present invention. These salts can be prepared on site during the manufacture of carriers or dosage form, or by separately reacting the purified compound of the present invention in free alkali form with a suitable organic or inorganic acid, and isolating the salt so formed during subsequent purification. Representative salts include hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, and succinate.

The formulations used in the present invention may also contain stabilizers, preservatives, buffers, antioxidants or other additives known to those skilled in the art. The use of such media and agents for pharmaceutically active substances is well known in the art.

As used herein, the terms "synergistic" and the like mean that the effect caused by a combination of two or more agents is greater than the additive effect of the two or more agents used alone. This synergistic effect of combination therapy comprises higher efficacy, lower side effects, or both. In some embodiments, the synergistic effect comprises a significant reduction in the side effects of the two therapeutic inhibitors due to dose reduction of the two therapeutic inhibitors, while the overall therapeutic efficacy remains at approximately the same or higher level. In some embodiments, the synergistic effect comprises a significant improvement in the efficacy of inhibiting cancer cell proliferation, while the side effects caused by the two drugs remain at approximately the same or lower level. The synergistic effect allows the use of individual drugs at lower doses to effectively treat the disease. In general, the synergistic combination of two or more drugs can lead to improvements in disease treatment compared to monotherapy.

Combination therapy allows the use of a lower dose of the first therapeutic agent, such as a PRMT5 inhibitor, or a lower dose of the second therapeutic agent, such as a CDK4/6 inhibitor, or lower doses of both therapeutic agents than would normally be required when either agent is used alone. The present invention encompasses any and all such "synergistic" effects.

The pharmaceutical composition may comprise a PRMT5 inhibitor and a CDK4/6 inhibitor for use in the methods of the present invention in a total amount of 0.01% to 99% by weight of the total composition, preferably 0.1% to 80% by weight of the total composition, and more preferably 0.1% to 50% by weight of the total composition. The weight ratio between the PRMT5 inhibitor and the CDK4/6 inhibitor may be in the range of 1:20 to 20:1, sometimes preferably 1:15 to 15:1, and sometimes more preferably 1:10 to 10:1.

For systemic administration, the daily dose of the PRMT5 inhibitor for adult treatment ranges from about 0.01 to about 150 mg/kg, preferably about 0.05 to about 100 mg/kg, and sometimes more preferably about 0.1 to about 50 mg/kg.

The pharmaceutical composition provided by the present invention can be used to treat and/or prevent various cancers, which may include or exclude the following cancers: glioblastoma multiforme, brain cancer, prostate cancer, pancreatic cancer, mantle cell lymphoma, non-Hodgkin's lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, cholangiocarcinoma, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, cutaneous squamous cell carcinoma, nasopharyngeal carcinoma, neuroblastoma, endometrial cancer, head and neck cancer, and cervical cancer.

In a preferred embodiment of the present invention, the cancer is metastatic cancer.

In a preferred embodiment of the present invention, the metastatic cancer is metastatic brain cancer.

### DESCRIPTION OF DRAWINGS

FIGS. 1A-1B: Antitumor inhibition curves and inhibition rates of compound A, Abemaciclib and their combination in the LU99 MTAP (-/-) deleted cell model
FIGS. 2A-2B: Antitumor inhibition curves and inhibition rates of compound C, Abemaciclib and their combination in the LU99 MTAP (-/-) deleted cell model

### SPECIFIC EXAMPLES

### 1. Test Compounds

The test compound Abemaciclib was developed by Eli Lilly, with the trade name Verzenio, and has the following chemical structure:

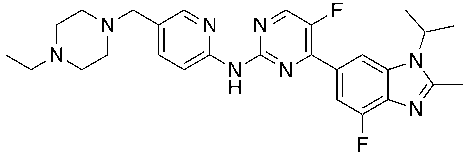

The chemical name of compound A used in this test is: (S)-4-amino-N-methyl-N-(6-(trifluoromethyl)-2,3-dihydrobenzofuran-3-yl)imidazo[1,5-a]quinoxaline-8-carboxamide, and it has the following chemical structure:

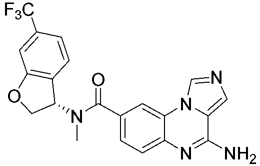

The chemical name of compound B used in this test is: 4-amino-N-cyclopropyl-7-fluoro-N-((5-(trifluoromethyl)pyridin-2-yl)methyl)imidazo[1,5-a]quinoxaline-8-carboxamide, and it has the following chemical structure:

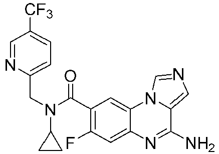

The chemical name of compound C used in this test is: (R)-4-amino-N-cyclopropyl-7-fluoro-*N*-(1-(5-trifluoromethyl)pyridin-2-yl)ethyl)imidazo[1,5-*a*]quinoxaline-8-carboxamide, and it has the following chemical structure:

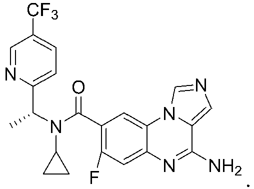

The chemical name of compound D used in this test is: (*S*)-4-amino-7-fluoro-*N*-methyl-*N*-(6-trifluoromethyl)-2,3-dihydrobenzofuran-3-yl)imidazo[1,5-*a*]quinoxaline-8-carboxamide, and it has the following chemical structure:

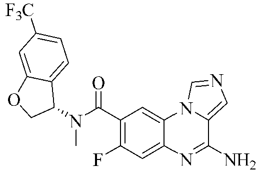

The chemical name of compound E used in this test is: (S)-4-amino-N-methyl-N-(6-(trifluoromethyl)-2,3-dihydrobenzofuran-3-yl)imidazo[1,5-a]pyrido[3,4-e]pyrazine-8-carboxamide, and it has the following chemical structure:

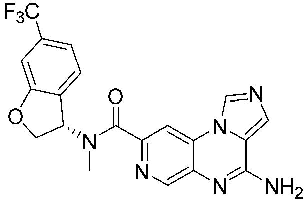

The chemical name of compound F used in this test is: (S)-4-amino-N-(methyl-d3)-N-(6-(trifluoromethyl)-2,3-dihydrobenzofuran-3-yl)imidazo[1,5-a]quinoxaline-8-carboxamide, and it has the following chemical structure:

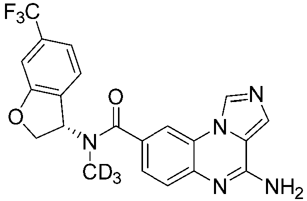

The chemical name of compound G used in this test is: (S)-4-amino-N-methyl-N-(6-(pentafluoro-λ6-sulfanyl)-2,3-dihydrobenzofuran-3-yl)imidazo[1,5-a]quinoxaline-8-carboxamide, and it has the following chemical structure:

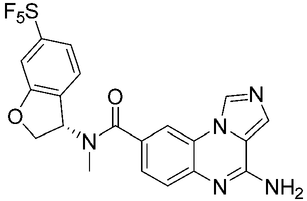

The chemical name of compound H used in this test is: (S)-4-amino-N-methyl-N-(6-(pentafluoroethyl)-2,3-dihydrobenzofuran-3-yl)imidazo[1,5-a]quinoxaline-8-carboxamide, and it has the following chemical structure:

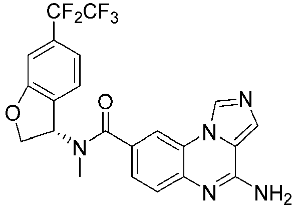

The chemical name of compound I used in this test is: (S)-amino-7-fluoro-N-methyl-N-(6-trifluoromethyl)-2,3-dihydrobenzofuran-3-yl)imidazo[1,5-a]quinoxaline-8-carboxamide, and it has the following chemical structure:

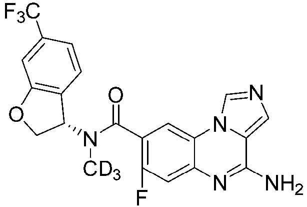

The chemical name of compound J used in this test is: (S)-4-amino-N-methyl-N-(6-(trifluoromethyl)-2,3-dihydrobenzofuran-3-yl)imidazo[1,5-a]quinoxaline-8-carboxamide-1-*d*, and it has the following chemical structure:

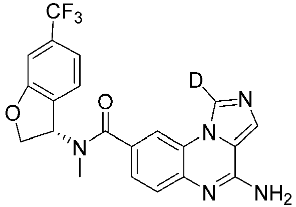

The chemical name of compound K used in this test is: (S)-4-amino-N-methyl-N-(6-(trifluoromethyl)-2,3-dihydrobenzofuran-3-yl)imidazo[1,5-a]quinoxaline-8-carboxamide-3-*d*, and it has the following chemical structure:

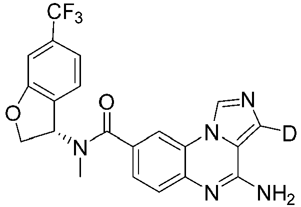

The chemical name of compound L used in this test is: (S)-4-amino-N-(methyl-d3)-N-(6-(trifluoromethyl)-2,3-dihydrobenzofuran-3-yl)imidazo[1,5-a]pyrido[3,4-e]pyrazine-8-carboxamide, and it has the following chemical structure:

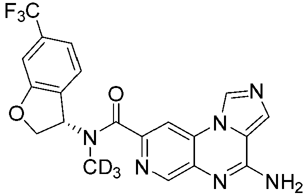

The chemical name of compound M used in this test is: (S)-4-amino-7-cyano-N-methyl-N-(6-(trifluoromethyl)-2,3-dihydrobenzofuran-3-yl)imidazo[1,5-a]quinoxaline-8-carboxamide, and it has the following chemical structure:

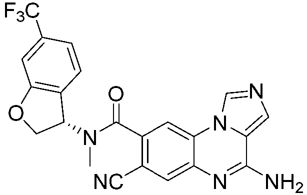

The chemical name of compound N used in this test is: (R)-4-amino-N-methyl-d3-N-(1-(5-trifluoromethyl)pyridin-2-yl)ethyl)imidazo[1,5-a]quinoxaline-8-carboxamide, and it has the following chemical structure:

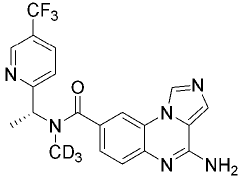

The chemical name of compound O used in this test is: (R)-4-amino-N-methyl-N-(1-(5-(trifluoromethyl)pyridin-2-yl)ethyl)imidazo[1,5-a]quinoxaline-8-carboxamide, and it has the following chemical structure: 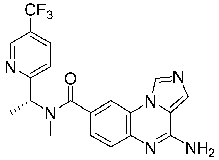
wherein, the preparation of compound A, compound D, compound E, compound F, compound G, compound H, compound I, compound J, compound K, compound L, and compound M is described in International Patent Application PCT/CN2023/120923;
wherein, the preparation of compound B, compound C, compound N and compound O is described in International Patent Application PCT/CN2023/111604,
which are incorporated herein by reference in their entirety, as if fully set forth herein.

### 2. Combination of Compounds in the LU99 MTAP (-/-) deleted cell model

### Cell Lines and Cell Culture:

The LU99 MTAP (-/-) cell line was purchased from Kyinno Biotechnology, Inc. The cells were cultured in a cell culture medium containing 90% RPMI1640 and 10% fetal bovine serum at 37°C and 5% CO₂. Only cells in the logarithmic growth phase were used in the experiments.

### Combination Test:

The cell density was adjusted using complete medium and cells were then seeded into a 96-well cell culture plate. The cells in the 96-well plate were incubated at 37°C and 5% CO₂. Two test compounds were added to the cell plate by serial dilution. After addition, the cells in the 96-well plate were incubated at 37°C and 5% CO₂ for 144 hours. The CTG reagent was thawed and the cell plate was equilibrated to room temperature for 30 minutes. An equal volume of CTG solution was added to each well. The plate was shaken on an orbital shaker for 5 minutes to lyse the cells. The cell plate was placed at room temperature for 20 minutes to stabilize the luminescence signal. The luminescence values were read and data were collected.

### Data Analysis:

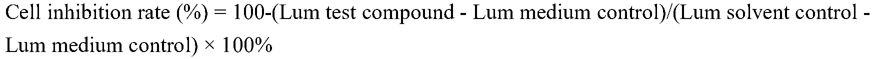

Cell inhibition rate (%) = 100-(Lum test compound - Lum medium control)/(Lum solvent control - Lum medium control) × 100%

Calcusyn was used to analyze the combination index (CI) values.

### Example 1: Test of compounds as single agents in the LU99 MTAP (-/-) deleted cell model

**Table 1**

| | **Max Inh%** | **IC₂₀ (nM)** | **IC₅₀ (nM)** |
|---|---|---|---|
| **Compound A** | 83.2 | 3.41 | 15.1 |
| **Compound C** | 82.4 | 3.64 | 13.4 |
| **Abemaciclib** | 94.3 | 49.9 | 58.7 |

Table 1 shows the IC₂₀ and IC₅₀ of each compound in the LU99 MTAP (-/-) deleted cells, as well as the maximum inhibition rate.

### Example 2: Test of compound A combined with Abemaciclib in the LU99 MTAP (-/-) deleted cell model

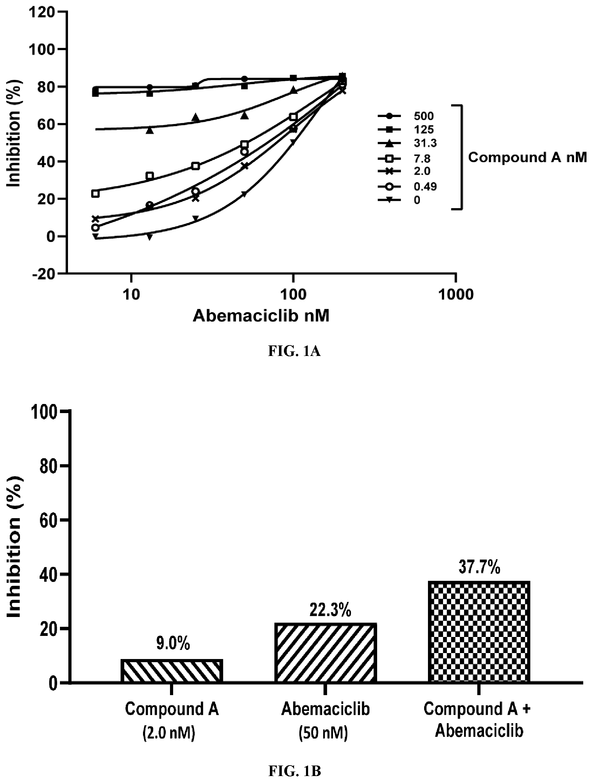

Regarding the test results for the combination of compound A and Abemaciclib in Lu99 MTAP(-/-) cells, the inhibition rate analysis is shown in Table 2, inhibition curves are shown in FIG. 1A, and inhibition rates are shown in FIG. 1B:

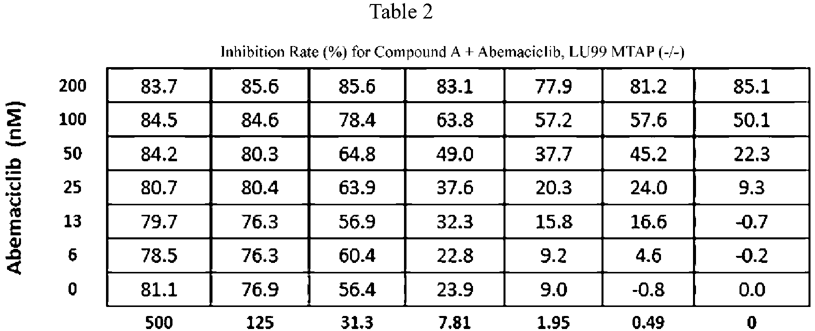

### Compound A (nM)

FIG. 1B shows the inhibition rates of compound A (2 nM) and Abemaciclib (50 nM) as single agents and in combination, indicating that under these conditions, the two compounds exhibit a synergistic effect.

### CI analysis:

**Table 3**

| CI for Compound A + Abemaciclib, LU99 MTAP (-/-) | | | | | | | **Abemaciclib (nM)** |
|---|---|---|---|---|---|---|---|
| | **2.42** | **1.20** | **0.98** | **1.01** | **1.17** | **1.05** | **200** |
| | **1.82** | **0.80** | **0.71** | **0.91** | **0.98** | **0.95** | **100** |
| | **1.62** | **0.75** | **0.76** | **0.78** | **0.82** | **0.63** | **50** |
| | **2.02** | **0.62** | **0.58** | **0.78** | **0.93** | **0.58** | **25** |
| | **2.11** | **0.74** | **0.68** | **0.79** | **0.90** | **0.45** | **13** |
| | **2.29** | **0.70** | **0.52** | **1.30** | **1.60** | **1.36** | **6** |
| Compound A (nM) | **500** | **125** | **31.3** | **7.81** | **1.95** | **0.49** | |

Table 3 shows the CI values for compound A with Abemaciclib.

### Example 3: Test of compound C combined with Abemaciclib in the Lu99 MTAP (-/-) deleted cell model

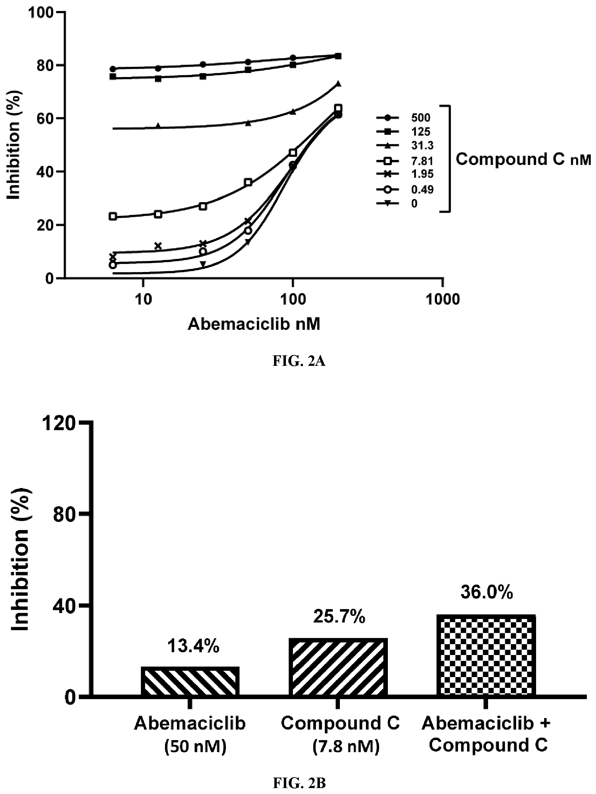

Regarding the test results for the combination of compound C and Abemaciclib in Lu99 MTAP(-/-) deleted cells, the inhibition rate analysis is shown in Table 4, inhibition curves are shown in FIG. 2A, and inhibition rates are shown in FIG. 2B.

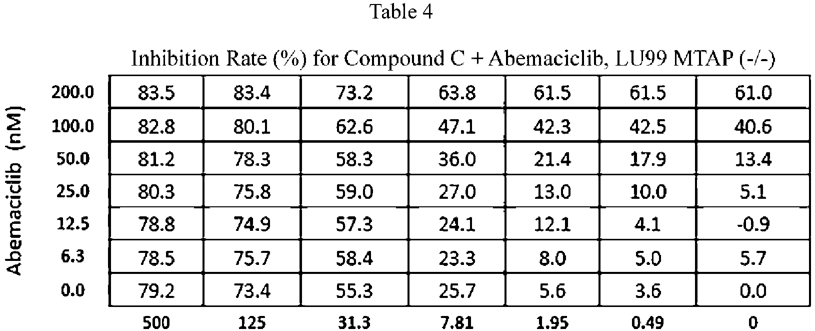

### Compound C (nM)

FIG. 2B shows the inhibition rates of Abemaciclib (50 nM) and compound C (7.8 nM) as single agents and in combination, indicating that under these conditions, the two compounds exhibit an additive effect.

### CI Analysis

**Table 5**

| CI for Compound C + Abemaciclib, LU99 MTAP (-/-) | | | | | | | **Abemaciclib (nM)** |
|---|---|---|---|---|---|---|---|
| | **1.56** | **0.57** | **0.58** | **0.68** | **0.68** | **0.66** | **200** |
| | **1.54** | **0.59** | **0.66** | **0.78** | **0.77** | **0.71** | **100** |
| | **1.69** | **0.58** | **0.62** | **0.83** | **1.18** | **1.24** | **50** |
| | **1.78** | **0.66** | **0.51** | **1.00** | **1.41** | **1.35** | **25** |
| | **1.99** | **0.67** | **0.51** | **1.01** | **1.10** | **2.29** | **13** |
| | **2.02** | **0.63** | **0.46** | **0.96** | **1.55** | **1.20** | **6** |
| Compound C (nM) | **500** | **125** | **31.3** | **7.81** | **1.95** | **0.49** | |

Table 5 shows the CI values for compound C with Abemaciclib.

Conclusion: Based on the inhibitory effects of the above compounds as single agents, the combination of compound A/compound C and Abemaciclib exhibits an additive effect.

Although preferred examples have been described hereinabove, it will be apparent to those skilled in the art that modifications may be made without departing from the present invention. Such modifications are considered as possible variants encompassed within the scope of the present invention.

## Claims

1. A pharmaceutical composition comprising a PRMT5 inhibitor having a first active substance and a CDK4/6 inhibitor compound having a second active substance, wherein the PRMT5 inhibitor having the first active substance has the structure of formula (I) or formula (II): 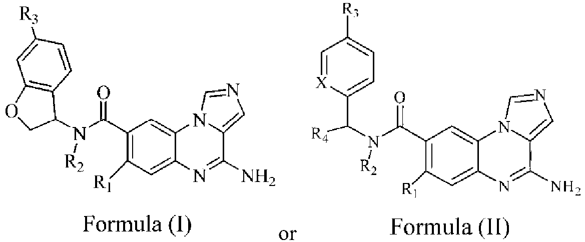
wherein, in formula (I) or formula (II),
R₁ is selected from H, halogen, C₁-C₆ alkyl, C₁-C₆ haloalkyl, and CN;
R₂ is selected from H, C₁-C₆ alkyl, C₁-C₆ haloalkyl, and C₃-C₆ cycloalkyl;
R₃ represents H, halogen, C₁-C₆ alkyl, C₁-C₆ haloalkyl, C₁-C₆ haloalkoxy, or SF₅;
in formula (II), R₄ represents hydrogen or C₁-C₆ alkyl; X represents CR₅ or N;
wherein, R₅ represents hydrogen, halogen, C₁-C₆ alkyl, C₁-C₆ haloalkyl, hydroxyl, -NH₂, or CN.

2. The pharmaceutical composition according to claim 1, wherein in the structure of formula (I) or formula (II):
R₁ is selected from hydrogen, halogen, C₁-C₆ alkyl, and C₁-C₆ haloalkyl;
R₂ is selected from hydrogen, C₁-C₆ alkyl, C₁-C₆ haloalkyl, and C₃-C₆ cycloalkyl;
R₃ represents hydrogen, halogen, C₁-C₆ haloalkyl, C₁-C₆ haloalkoxy, or SF₅;
R₄ represents hydrogen or methyl;
in formula (II), X represents CH or N.

3. The pharmaceutical composition according to claim 1, wherein R₁ is selected from hydrogen or fluorine.

4. The pharmaceutical composition according to claim 1, wherein R₂ is selected from cyclopropyl or methyl.

5. The pharmaceutical composition according to claim 1, wherein R₃ represents CF₃.

6. The pharmaceutical composition according to claim 1, wherein R₄ represents hydrogen or methyl.

7. The pharmaceutical composition according to claim 1, wherein X represents N.

8. The pharmaceutical composition according to claim 1, wherein the PRMT5 inhibitor having the first active substance is selected from the following compounds or any combination thereof:

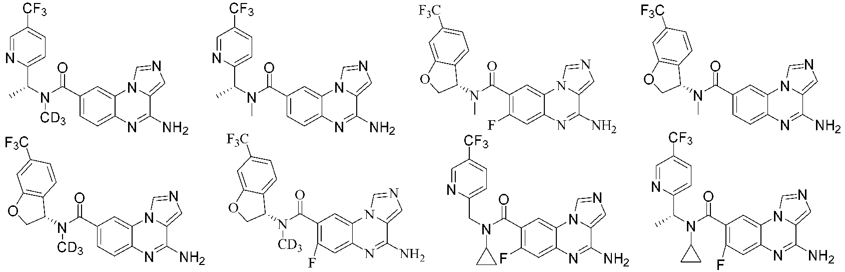

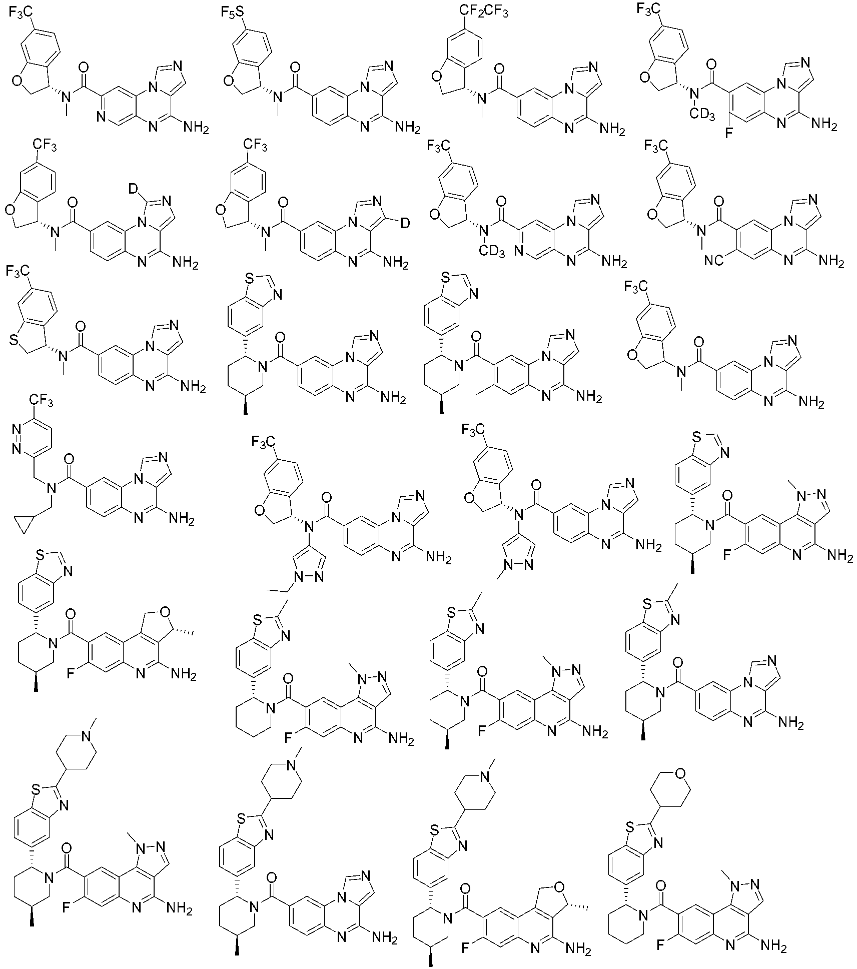

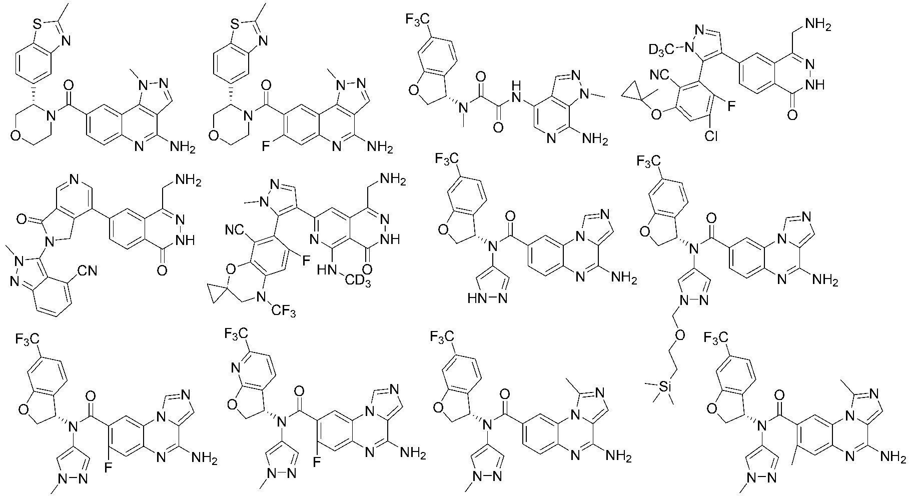

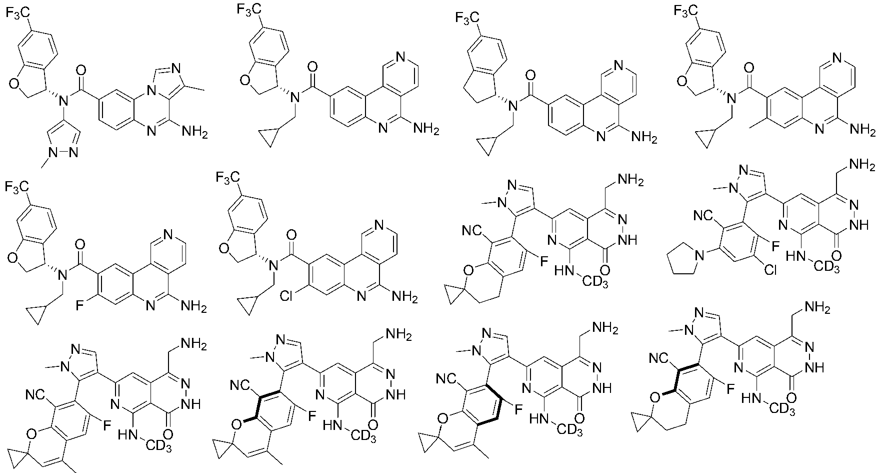

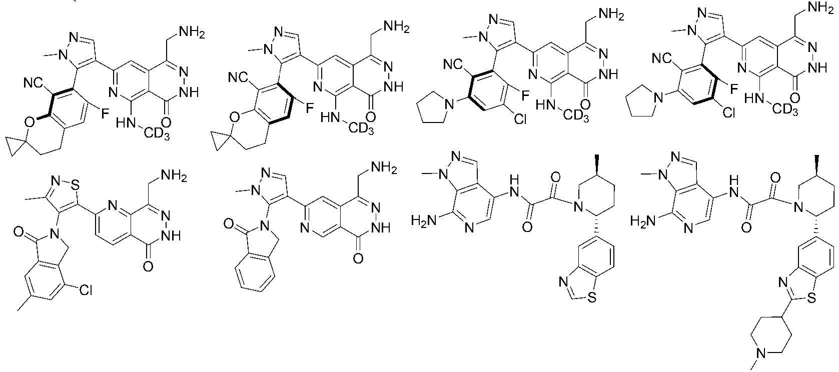

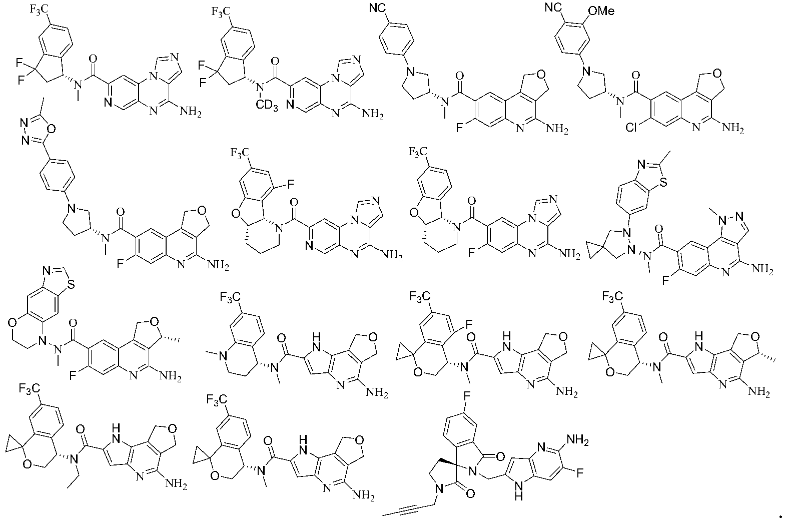

9. The pharmaceutical composition according to any one of claims 1-8, wherein the CDK4/6 inhibitor having the second active substance is selected from any one of the following compounds or a pharmaceutically acceptable salt thereof: Palbociclib (Ibrance^{®}), Abemaciclib (Verzenio^{®}), Ribociclib (Kisqali^{®}), Trilaciclib (Cosela^{™}), PF-06873600 (CAS: 2185857-97-8) or any combination thereof.

10. The pharmaceutical composition according to claim 9, wherein the CDK4/6 inhibitor having the second active substance is selected from Palbociclib (Ibrance^{®}), Abemaciclib (Verzenio^{®}), Ribociclib (Kisqali^{®}), Trilaciclib (Cosela^{™}) or a pharmaceutically acceptable salt thereof.

11. The pharmaceutical composition according to claim 9, wherein the CDK4/6 inhibitor having the second active substance is selected from Abemaciclib (Verzenio^{®}) or a pharmaceutically acceptable salt thereof.

12. A method for treating cancer or a tumor, comprising administering the pharmaceutical composition according to any one of claims 1-11 to an individual in need.

13. The method according to claim 12, wherein the tumor or cancer is selected from: glioblastoma multiforme, brain cancer, prostate cancer, pancreatic cancer, mantle cell lymphoma, non-Hodgkin's lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, cholangiocarcinoma, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, cutaneous squamous cell carcinoma, nasopharyngeal carcinoma, neuroblastoma, endometrial cancer, head and neck cancer, and cervical cancer.

14. The method according to claim 12, wherein the cancer is metastatic cancer.

15. The method according to claim 14, wherein the metastatic cancer is metastatic brain cancer.
